(19)

Europäisches
Patentamt
European
Patent Office
Office européen
des brevets

(11) **EP 2 422 849 A1**

(12) **EUROPEAN PATENT APPLICATION**

(43) Date of publication:
   **29.02.2012 Bulletin 2012/09**

(21) Application number: **10173258.4**

(22) Date of filing: **18.08.2010**

(51) Int Cl.:
   **A61P 35/00** (2006.01)    **A61K 31/404** (2006.01)
   **A61K 31/353** (2006.01)    **A61K 31/352** (2006.01)

(84) Designated Contracting States:
   **AL AT BE BG CH CY CZ DE DK EE ES FI FR GB
   GR HR HU IE IS IT LI LT LU LV MC MK MT NL NO
   PL PT RO SE SI SK SM TR**
   Designated Extension States:
   **BA ME RS**

(71) Applicant: **Grindeks, a joint stock company**
   **1057 Riga (LV)**

(72) Inventors:
   • **Kalvins, Ivars**
     **Riga 1006 (LV)**

   • **Stonans, Ilmars**
     **Riga 1057 (LV)**
   • **Sestakova, Irina**
     **Riga 1006 (LV)**
   • **Domracova, Ilona**
     **Riga 1006 (LV)**
   • **Jascenko, Elina**
     **Riga 1006 (LV)**
   • **Bridane, Veronika**
     **Riga 1006 (LV)**
   • **Kanepe, Iveta**
     **Riga 1006 (LV)**

(54) **Composition of Indole-3-carbinol and either catechin, kaempherol, myricetin or luteolin for potentiating antitumour effect and for treating tumours**

(57)    Novel pharmaceutical composition of treating cancer, preferably malignant cancer by way of enteral use have been described, which contain Indole-3-carbinol and one of natural flavonoid derivative, preferably (+)-Catechin, Kaempferol, Myricetin or Luteolin.

EP 2 422 849 A1

**Description**

Technical Field

The present invention relates to a novel composition of Indole-3-carbinol and natural flavonoid derivatives, in particular, it relates to the unexpected synergism of a combination of Indole-3-carbinol (13C) and one of natural flavonoid derivative in the treatment of cancer; and to processes for preparing the composition and its use for chemotherapy of tumours, especially malignant tumours.

Background Art

More than 11 million people are diagnosed with cancer every year. It is estimated that there will be 16 million new cases every year by 2020. Cancer causes 7 million deaths every year - or 12.5% of deaths worldwide.

Therefore, a great number of investigations, developments and research work have been carried out to find anticancer agents, which can be used in chemotherapy of malignant tumours. There are known various anticancer agents and results of cancer treatment improve with every year, nevertheless the amount of effective dose is still high.

In nowadays a big role in cancer experiments and in treating cancer take natural compounds. Phytochemicals are one of them, which are naturally occurring anticancer agents.

Phytochemicals are naturally occurring biochemical's that plants developed in order to protect themselves from oxidation, insects, disease organisms and other hazards in their environment, published in http://www.suite101 .com/ articie.cfm/new_cancer_treatments/104912 18.07.2007

There are more than thousand phytochemicals. Some of such are phenolic compounds - derivative of glucosinolate family such as Indole-3-carbinol and natural flavonoid derivatives, such as (+)-Catechin, Kaempferol, Myricetin, Luteolin.

Indole-3-carbinol (13C) is a breakdown product of the glucosinolate glucobrassicin, also known as indole-3-glucosinolate. Glucosinolates are beta-thioglucoside N-hydroxysulfates, which are primarily found in cruciferous vegetables (cabbage, broccoli sprouts, Brussels sprouts. Indole-3-carbinol may have cancer chemopreventive activity, it may also have anticarcinogenic, antioxidant and anti-atherogenic activities, see http://www.pdrhealt.com/drug info/nmdrugprofiles/nutsupdrugs/ind-0315.s html 11.07.2007

Because of its anticarcinogenic effects in experimental animals and humans, In has received special attention as a possible chemopreventive agent. In has also been found to inhibit the growth of various cancer cells and possibly to inhibit breast cancer invasion and migration (see Sarkar FH, Li Y. "Indole-3-carbinol and prostate cacner", published in "The Journal of Nutrition" 2004, vol.134, No 12, p.3493S-3498S.

Indole-3-carbinol acts as an inhibitor of Akt and nuclear factor kB (NF-{kappa} B), which plays important roles in cell survival and which are believed to be potential targets in cancer therapy. Studies have already shown that the inactivation of Akt and NF- {kappa} B is responsible for chemosensitization of chemoresistant cancer cells, See (F.Sarkar, Y.Li "Indole-3-Carbinol and Prostate Cancer", published in "The American Society for Nutritional Sciences" 2004 vol. 134, p.3493S-3498S).

Flavonoids are polyphenolic secondary metabolites widely dispersed throughout the plant kingdom and found in substantial levels in commonly consumed fruits, vegetables and beverages. Flavonoids, which are benzo-{gamma}-pyrone derivatives with A, B and C rings, are categorised as:

flavonols, flavones, flavan-3-ols, isoflavones, flavonones and anthocyanidins. They have been shown to possess a variety of biological activity at non-toxic concentrations in organisms. The role of dietary flavonoids in cancer prevention is widely discussed. Compelling data from laboratory studies, epidemiological investigations, and human clinical trials indicate that flavonoids have important effect on cancer chemoprevention and chemotherapy. Many mechanisms of action have been identified, including carcinogen inactivation, antiproliferation, cell cycle arrest, induction of apoptosis and differentiation, inhibition of angiogenesis, antioxidation and reversal of multidrug resistance or a combination of these mechanisms. Based on these results, flavonoids may be promising anticancer agents, see (REN W., QIAO Z., "Flavonoids: Promising anticancer agents", published in "Medical Research Reviews" 2003, vol. 23., no.4., p.519-534).

Catechins are a class of flavonoids with potent antioxidant and cancer chemopreventive properties. These compounds are found in a variety of plants and are present in particularly high amounts in tea leaves, where they may constitute up to 30% of dry leaf weight. High levels of monomeric (+)-catechin are found in the skin and seeds of fruits such as apples and grapes. In keeping with ability of phenolic antioxidants to induce the expression of phase II detoxifying enzymes in mammalian cells. (+)-catechin produces antimutagenic effects, see (MICHAEL J., "(+)-Catechins Inhibits Intestinal Tumour Formation and Suppresses Focal Adhesion Kinase Activation in the Min/+Mouse", published in "Cancer Research" 2001, vol.61, no.1, p.118-125).

Myricetin is a naturally-occurring flavonoid in many grapes, berries, fruits, vegetables, herbs, as well as other plants. Myricetin is considered to be an antioxidant, which means that it is capable eliminating cancer causing free radicals with in the body, published in http://nutritional-supplement-guides.com/Myricetin.html 25.07.2007

There are data that myricetin exhibits a significant induction of differentiation in the human osteoblast-like cell line MG-63, and Myricetin affects inflammatory cytokines-mediated apoptosis in osteoblast cells. Treatment of MG-63 cells with myricetin not only inhibited anti-Fas IgM-induced apoptosis, but also blocked the synergetic effect of anti-Fas IgM with Tumor Necrosis factor-alpha or IL-1 beta on cell death, see (KUO PL, "Myricetin inhibits the induction of anti-Fas IgM-, tumor necrosis factor-alpha- and interleukin-1-beta-mediated apoptosis by Fas pathway inhibition in human osteoblast cell line MG-63.", published in "Life Sciences" 2005, vol.77., no.23., p.2964-76.)

Kaempferol is a natural flavonoid which has been isolated from tea, broccoli, Delphinium, grapefruit and other plant sources. Kaempferol is a strong antioxidant and helps to prevent oxidative damage of our cells, lipids and DNA. Studies have also confirmed that kaempferol acts as a chemopreventive agent, which means that it inhibits the formation of cancer cells, published in http://www.phytochemicals.info/phytochemicals/kaempferol.php 26.07.2007

Disclosure of invention

Technical problem

In malignant tumour treatment different kind of pharmaceutical compositions are used, including compositions of different natural compounds, but just only partial remission of malignant tumour was obtained.

The further aim of present invention is to developed effective pharmaceutical composition that can be used in malignant tumour treatment for achieving increased remission of tumours. Technical solution

The present invention is directed to a composition for treating a tumour, especially malignant tumour in an individual who need such treatment, comprising the administration to said individual of a pharmacologically effective dose of Indole-3-Carbinol and one or more natural flavonoid derivatives, especially (+)-Catechin, Myricetin, Kaempferol or Luteolin.

While attempting to develop a pharmaceutical composition for malignant tumour treatment having an essentially lower toxicity, we unexpectedly found that doses adequate to the therapeutic ones used in the clinical use of pharmaceutical compositions containing Indole-3-carbinol, the coadministration or combined use in pharmaceutical compositions of a compound of a natural flavonoid derivatives, preferably (+)-Catechin, Myricetin, Kaempferol or Luteolin leads to advantageous effects, such as:

- The combination of Indole-3-carbinol (In) and natural flavonoid derivative, preferably (+)-Catechin, Kaempferol, Myricetin or Luteolin showed remarkable synergism between the components in the treatment of tumour;
- The use of a combination of 13C and natural flavonoid derivative, preferably (+)-Catechin, Myricetin, Kaempferol or Luteolin, allows efficacy increase growth inhibition of S-180 ascites tumour.

There is no particular restriction on the unit dosage from which can be adopted the antitumour composition of the invention in the treatment of malignant tumours in mammals. The unit dosage form is selected according to the purpose of treatment. Examples thereof are oral dosage form such as tablets, coated tablets, pills, powders, granules, capsules, solutions, suspensions, emulsions, etc.; and parenteral dosage forms such as suppositories, ointments, plasters, etc. These dosage forms can be manufactured by conventional pharmaceutical procedures known in this field.

As the carrier for shaping into the form of tablets, there can be employed various excipients such as lactose, sucrose, sodium chloride, glucose, urea, starch, calcium carbonate, kaolin, crystalline cellulose, silicic acid, etc., binders such as simple syrup, glucose solution, starch solution, gelatine solution, carboxymethylcellulose, shellac, methylcellulose, potassium phosphate, polyvinylpyrrolidone, etc.; disintegrators such as fry starch, sodium alginate, agar powder, laminaran powder, sodium hydrogen carbonate, calcium carbonate, polyoxyethylene-sorbitan fatty acid esters, sodium lauryl sulphate, stearic acid monoglyceride, starch, lactose, etc.; antidisintegrators such as sucrose, stearic acid, cacao butter, hydrogenated oil, etc.; absorption promoters such as quaternary ammonium bases, sodium lauryl sulphate, etc.; humectants such as glycerol, starch, etc.; adsorbents such as starch, lactose, kaolin, bentonite, colloidal silicic acid, etc.; and lubricants such as purified talc, steric acid salts, boric acid powder, polyethylene glycol, etc. Where necessary, the tablets may be in the form of coated tablets such as sugar-coated tablets gelatin-coated tablets, enteric-coated tablets, film-coated tablets, or double or multi-layer tablets, etc.

The carrier for shaping into the form of pills includes, for example, various excipients such as glucose, lactose, starch, cacao butter, hardened vegetable oil, kaolin, talc, etc.; binders such as gum Arabic powder, gum tragacanth powder, gelatine, etc.; and disintegrators such as laminaran, agar, etc.. The carrier for shaping into the form of suppositories includes, for example polyethylene glycol, cacao butter, higher alcohols, esters of higher alcohols, gelatin, semi-synthetic glycerides, etc.

Capsules are manufactured by mixing the Indole-3-carbinol with natural flavonoid derivative, preferably (+)-Catechin, Myricetin, Kaempferol or Luteolin, with any of the carriers mentioned above and encapsulating the mixture in hard gelatine capsule, soft capsule or other capsules.

For manufacturing in the form of pastes, creams and gels, there is employed a diluent such as, for example, white petrolatum, paraffin, glycerol, cellulose derivatives, polyethylene glycols, silicone, bentonite, etc.

Best mode for carrying out the invention

The present invention in the following will be described in more detail with reference to pharmacological tests and examples illustrating the preparation of the antitumour effect-potentiating compositions of the invention comprising Indole-3-carbinol and natural flavonoid derivative, preferably (+)-Catechin, Myricetin, Kaempferol or Luteolin. Mode(s) for Carrying Out Invention

Experimental example

Experiments in vivo were carried out on mice sarcoma S-180 ascites form.

The anti-tumour activity of the pharmaceutical composition of Indole-3-carbinol and natural flavonoid derivative was determined by introducing them into ICR mice of initial weight 20-23 g during a fortnight. Throughout the experiment, the mice were kept under standard laboratory conditions at a temperature of $22\pm2$ °C, relative humidity 55 $\pm$15%, ventilation - 15-20 changes of air amount/hour and a 12 hours light/darkness cycle, and were fed with standard laboratory animal food.

Mice sarcoma S-180 transplanted intraperitoneally to ICR mice in amount $5 \times 10^6$ cell/mouse.

Then, 24 hours later, orally administration of compounds was started once a day at the 1st, 3rd, 5th, 7th, 9th, 11th and 13th days.

The compound efficacy was expressed as the percentage of growth inhibition of S-180 ascites tumour, calculated using the equation:

Growth inhibition of S - -180 ascites tumour(%) $= 100 - \left( \dfrac{T}{C} \times 100 \right)$

wherein C - mean weight (g) of the control group and T - mean weight (g) of the experimental group.

The results of Table 1 show the growth inhibition of S-180 ascites tumour at 13th day after the transplantation of tumour in mice.

Table 1

| Nr. | Drug | Dosage (mg/kg) | Growth inhibition of S-180 ascites tumour, % |
|---|---|---|---|
| 1 | Indole-3-carbinol | 75 | -13 |
| 2 | Catechin | 300 | 6 |
| 3 | Indole-3-carbinol+ Catechin | 75+300 | 47 |
| 4 | Indole-3-carbinol | 200 | -15 |
| 5 | Kaempferol | 100 | -9 |
| 6 | Indole-3-carbinol+Kaempferol | 200+100 | 67 |
| 7 | Indole-3-carbinol | 200 | -15 |
| 8 | Myricetin | 200 | -4 |
| 9 | Indole-3-carbinol+Myricetin | 200+200 | 40 |
| 10 | Indole-3-carbinol | 200 | -15 |
| 11 | Luteolin | 200 | -39 |
| 12 | I ndole-3-carbinol+Luteolin | 200+200 | 85 |

As it is shown in Table 1 combinations of Indole-3-carbinol and one of natural flavonoid derivative showed remarkable synergism between the components and allows efficacy increase growth inhibition of S-180 ascites tumour.

Formulation:Granules

|  | Example 1 | Example 2 | Example 3 | Example 4 |
|---|---|---|---|---|
| I3C | 400 mg | 200 mg | 400 mg | 20 mg |
| Catechin | 400 mg | 0 | 0 | 0 |
| Kaempferol | 0 | 400 mg | 0 | 0 |
| Luteolin | 0 | 0 | 0 | 200 mg |
| Myricetin | 0 | 0 | 100 mg | 0 |
| Lactose | 200 mg | 200 mg | 200 mg | 200 mg |
| Corn starch | 200 mg | 200 mg | 200 mg | 200 mg |
| Hydroxumethylcellulose | 10 mg | 10 mg | 10 mg | 10 mg |
| Total | 1210 mg | 1010 mg | 910 mg | 630 mg |

Using the conventional procedure, the granules were prepared according to the above formula.
Formulation: Tablets

|  | Example 1 | Example 2 | Example 3 | Example 4 |
|---|---|---|---|---|
| 13C | 50 mg | 100 mg | 10 mg | 100 mg |
| Catechin | 50 mg | 0 | 0 | 0 |
| Kaempferol | 0 | 0 | 120 mg | 0 |
| Myricetin | 0 | 10 mg | 0 | 0 |
| Luteolin | 0 | 0 | 0 | 50 mg |
| Lactose | 100 mg | 100 mg | 100 mg | 100 mg |
| Mg stearate | 10 mg | 10 mg | 10 mg | 10 mg |
| Talc | 10 mg | 10 mg | 10 mg | 10 mg |
| Total | 270 mg | 280 mg | 300 mg | 320 mg |

Using the conventional procedure, the tablets were prepared according to the above formula.
Formulation: Capsules

|  | Example 1 | Example 2 | Example 3 | Example 4 |
|---|---|---|---|---|
| I3C | 90 mg | 100 mg | 10 mg | 50 mg |
| Catechin | 30 mg | 0 | 0 | 0 |
| Kaempferol | 0 | 50 mg | 0 | 0 |
| Myricetin | 0 | 0 | 110 mg | 0 |
| Luteolin | 0 | 0 | 0 | 200 mg |
| Lactose | 100 mg | 100 mg | 100 mg | 150 mg |
| Talc | 100 mg | 100 mg | 100 mg | 150 mg |
| Ca stearate | 7 mg | 7 mg | 7 mg | 7 mg |
| Aerosil | 3 mg | 3 mg | 3 mg | 5 mg |
| Total | 330 mg | 360 mg | 330 mg | 560 mg |

Using the conventional procedure, the capsules were prepared according to the above formula.
Formulation: Suppository

|  | Example 1 | Example 2 | Example 3 | Example 4 |
|---|---|---|---|---|
| 13C | 50 mg | 200 mg | 100 mg | 20 mg |
| Catechin | 150 mg | 0 | 0 | 0 |
| Kaempferol | 0 | 100 mg | 0 | 0 |
| Myricetin | 0 | 0 | 100 mg | 0 |
| Luteolin | 0 | 0 | 0 | 200 mg |
| Witepsol W-35 | 350 mg | 400 mg | 300 mg | 380 mg |
| Total | 500 mg | 700 mg | 500 mg | 600 mg |

Using the conventional procedure, the suppositories were prepared according to the above formula.

Formulation: Suspension

|  | Example 1 | Example 2 | Example 3 | Example 4 |
|---|---|---|---|---|
| 13C | 150 mg | 200 mg | 100 mg | 180 mg |
| Catechin | 150 mg | 0 | 0 | 0 |
| Kaempferol | 0 | 50 mg | 0 | 0 |
| Myricetin | 0 | 0 | 200 mg | 0 |
| Luteolin | 0 | 0 | 0 | 90 mg |
| Glycerin | 100 mg | 100 mg | 100 mg | 100 mg |
| Disstilled water | 200 mg | 150 mg | 200 mg | 150 mg |
| Total | 600 mg | 500 mg | 600 mg | 520 mg |

Using the conventional procedure, the suppositories were prepared according to the above formula.

Formulation: Parenteral solution

|  | Example 1 | Example 2 | Example 3 | Example 4 |
|---|---|---|---|---|
| 13C | 250 mg | 400 mg | 100 mg | 150 mg |
| Catechin | 125 mg | 0 | 0 | 0 |
| Myricetin | 0 | 100 mg | 0 | 0 |
| Kaempferol | 0 | 0 | 300 mg | 0 |
| Luteolin | 0 | 0 | 0 | 200 mg |
| Sodium carbonate | 500 mg | 500 mg | 500 mg | 500 mg |
| Sodium hydroxide | 80 mg | 80 mg | 80 mg | 80 mg |
| Distilled water | (suitable amounts) | (suitable amounts) | (suitable amounts) | (suitable amounts) |

Using the conventional procedure, the parenteral solutions were prepared according to the above formula.

Industrial Application

According to the invention, the pharmaceutical composition of treating malignant tumour which contains Indole-3-carbinol and one of natural flavonoid derivative, preferably (+)-Catechin, Kaempferol, Myricetin or Luteolin in clinically efficacious amount might be manufactured in a standard pharmaceutical plant.

**Claims**

1. A pharmaceutical composition comprising Indole-3-carbinol (I3C) or a pharmaceutically acceptable salt thereof and one of natural flavonoid derivative, selected from the group of (+)-Catechin, Kaempferol, Myricetin or Luteolin.

2. A pharmaceutical composition according to claim 1, wherein one of natural flavonoid derivative is (+)-Catechin.

3. A pharmaceutical composition according to claim 1, wherein one of natural flavonoid derivative is Kaempferol.

4. A pharmaceutical composition according to claim 1, wherein one of natural flavonoid derivative is Myricetin.

5. A pharmaceutical composition according to claim 1, wherein one of natural flavonoid derivative is Luteolin.

6. A pharmaceutical composition according to claim 1, wherein the preferable ratio of the active ingredients Indole-3-carbinol and one of natural flavonoid derivative, selected from the group of (+)-Catechin, Kaempferol, Myricetin or Luteolin, is from 1:100 to 100:1.

7. A pharmaceutical composition according to claim 1, wherein the preferable ratio of the active ingredients Indole-3-carbinol and one of natural flavonoid derivative, selected from the group of (+)-Catechin, Kaempferol, Myricetin or Luteolin, is from 1:25 to 25:1.

8. A pharmaceutical composition according to claim 1, wherein the preferable ratio of the active ingredients Indole-3-carbinol and one of natural flavonoid derivative, selected from the group of (+)-Catechin, Kaempferol, Myricetin or Luteolin, is from 2:1 to 1:2.

9. Use of pharmaceutical composition according to any preceding claim as a medicament.

10. A pharmaceutical composition according to claims 1-8 for use in the treatment of a tumour.

11. Use of pharmaceutical composition according to claims 1-8 for the manufacture of a medicament for administration simultaneously, sequentially or separately for treatment and/or prevention of tumour.

Europäisches
Patentamt

European
Patent Office

Office européen
des brevets

# EUROPEAN SEARCH REPORT

**Application Number**

EP 10 17 3258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FAN S ET AL: "BRCA1 and BRCA2 as molecular targets for phytochemicals indole-3-carbinol and genistein in breast and prostate cancer cells", BRITISH JOURNAL OF CANCER, NATURE PUBLISHING GROUP, LONDON, GB, vol. 94, no. 3, 1 February 2006 (2006-02-01), pages 407-426, XP002506760, ISSN: 0007-0920, DOI: DOI:10.1038/SJ.BJC.6602935 [retrieved on 2006-01-24] * abstract * | 1-11 | INV.<br>A61P35/00<br>A61K31/404<br>A61K31/353<br>A61K31/352 |
| A | POUGET C ET AL: "FLAVONOIDS: STRUCTURAL REQUIREMENTS FOR ANTIPROLIFERATIVE ACTIVITY ON BREAST CANCER CELLS", BIOORGANIC & MEDICINAL CHEMISTRY LETTERS, PERGAMON, ELSEVIER SCIENCE, GB, vol. 11, no. 24, 1 January 2001 (2001-01-01), pages 3095-3097, XP009058137, ISSN: 0960-894X * page 3097, left-hand column, paragraph 3 - right-hand column, paragraph 1 * | 1-11 | |
| A | HOWELLS LYNNE M ET AL: "Predicting the physiological relevance of in vitro cancer preventive activities of phytochemicals.", ACTA PHARMACOLOGICA SINICA SEP 2007, vol. 28, no. 9, September 2007 (2007-09), pages 1274-1304, XP002506708, ISSN: 1671-4083 * page 1279, right-hand column, paragraph 3 - page 1280, left-hand column, paragraph 1 * | 1-11 | |

TECHNICAL FIELDS
SEARCHED (IPC)

A61K
A61P

-/--

The present search report has been drawn up for all claims

| Place of search | Date of completion of the search | Examiner |
|---|---|---|
| Munich | 11 March 2011 | Herrera, Suzanne |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 10 17 3258

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DEORUKHKAR A ET AL: "Back to basics: How natural products can provide the basis for new therapeutics", EXPERT OPINION ON INVESTIGATIONAL DRUGS 200711 GB, vol. 16, no. 11, November 2007 (2007-11), pages 1753-1773, XP002506709, ISSN: 1354-3784 * page 1763, right-hand column, paragraph 3 - page 1764, left-hand column, paragraph 2 * | 1-11 | |
| A | SCHMOLL H-J: "DIHYDROPYRIMIDINE DEHYDROGENASE INHIBITION AS A STRATEGY FOR THE ORAL ADMINISTRATION OF 5-FLUOROURACIL: UTILITY IN THE TREATMENT OF ADVANCED COLORECTAL CANCER", ANTI-CANCER DRUGS, RAPID COMMUNICATIONS, OXFORD, vol. 14, no. 9, 1 January 2003 (2003-01-01), pages 695-702, XP009062217, ISSN: 0959-4973 * abstract * | 1-11 | |

TECHNICAL FIELDS
SEARCHED      (IPC)

The present search report has been drawn up for all claims

| Place of search | Date of completion of the search | Examiner |
|---|---|---|
| Munich | 11 March 2011 | Herrera, Suzanne |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 2 422 849 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SARKAR FH ; LI Y.** Indole-3-carbinol and prostate cacner. *The Journal of Nutrition,* 2004, vol. 134 (12), 3493S-3498S
- **F.SARKAR ; Y.LI.** Indole-3-Carbinol and Prostate Cancer. The American Society for Nutritional Sciences, 2004, vol. 134, 3493S-3498S
- **REN W. ; QIAO Z.** Flavonoids: Promising anticancer agents. *Medical Research Reviews,* 2003, vol. 23 (4), 519-534
- **MICHAEL J.** +)-Catechins Inhibits Intestinal Tumour Formation and Suppresses Focal Adhesion Kinase Activation in the Min/+Mouse. *Cancer Research,* 2001, vol. 61 (1), 118-125
- **KUO PL.** Myricetin inhibits the induction of anti-Fas IgM-, tumor necrosis factor-alpha- and interleukin-1-beta-mediated apoptosis by Fas pathway inhibition in human osteoblast cell line MG-63. *Life Sciences,* 2005, vol. 77 (23), 2964-76